# EUROPÄISCHE PATENTSCHRIFT

(11) **EP 1 632 776 B1**
(45) Veröffentlichungstag und Bekanntmachung des Hinweises auf die Patenterteilung: **04.06.2008**
(21) Anmeldenummer: 05450147.3
(22) Anmeldetag: 01.09.2005
(51) Int. Cl.: G01N 33/49, G01N 27/416, G01N 7/00

(54) **Verfahren zur Detektion einer Gasblase in einer wässrigen Flüssigkeit**
Method of detecting a gas bubble in an aqueous liquid
Procédé de détection d'une bulle gazeuse dans un liquide aqueux

(30) Priorität: 02.09.2004 AT 14682004
(43) Veröffentlichungstag der Anmeldung: 08.03.2006
(73) Patentinhaber: F. Hoffmann-La Roche AG, 4070 Basel (CH); Roche Diagnostics GmbH, 68305 Mannheim (DE)
(72) Erfinder: Kaltenbeck, Heinz, 8010 Graz (AT); Grübler, Robert, 8042 Graz (AT); Landschützer, Egon, 8051 Graz (AT)
(74) Vertreter: Babeluk, Michael

(56) Entgegenhaltungen:
- EP-A- 0 355 896
- EP-A2- 1 084 963
- AT-A- 12 502 003
- US-A1- 2003 080 002

## Beschreibung

Die Erfindung betrifft ein Verfahren zur Detektion einer Gasblase, vorzugsweise einer Luftblase, in einer wässrigen Flüssigkeit, vorzugsweise einer Kalibrier-, Kontroll- oder Probenflüssigkeit, welche Gasblase sich in zumindest teilweisem Kontakt mit dem sensitiven Bereich eines in einer Messkammer angeordneten Sensors zur Bestimmung der Konzentration einer in der Flüssigkeit gelösten Gaskomponente befindet.

In der klinischen Diagnostik werden Analysesysteme zur Blutgasanalyse oder zu sonstigen Messungen an in flüssiger Form vorliegenden Proben verwendet. Solche Systeme werden beispielsweise zur Bestimmung des Sauerstoff- oder Kohlendioxidpartialdrucks von Blut, der Hämoglobinparameter von Vollblut sowie des pH-Werts oder der Konzentration von Ionen oder speziellen Metaboliten eingesetzt.

Solche komplexen Analysesysteme besitzen zur Bestimmung der jeweiligen Parameter häufig verschiedene sensorische Elemente, welche für viele Bestimmungen eingesetzt werden. Solche sensorischen Elemente sind beispielsweise elektrochemische oder optische Sensoren zur Bestimmung der Gaswerte, des pH-Wertes, der Ionenwerte und der Metabolitwerte oder optische Messeinheiten zur Bestimmung der Hämoglobinwerte.

Die US 3,874,850 A (Sorensen et al.) beschreibt ein solches Analysesystem zur Blutanalyse. Dieses System weist mehrere sensorische Elemente auf, welche zur Bestimmung des pH-Wertes, der Partialdrücke von Sauerstoff und Kohlendioxid und des Hämoglobingehaltes einer Blutprobe dienen. Diese sensorischen Elemente sind durch ein komplexes fluidisches System aus verschiedensten fluidischen Bauteilen wie Schläuchen, Vorrats- und Abfallbehältern, Pumpen oder Ventilen verbunden, wobei bei diesem System die Probe mit Hilfe von Spritzen oder Kapillaren vom Patienten zum Analysesystem transportiert wird.

Bei elektrochemischen Gassensoren, auch gasselektive oder gassensitive Elektroden genannt, diffundieren die zu bestimmenden Gasmoleküle durch eine gasdurchlässige und weitgehend flüssigkeits- und ionenundurchlässige Membran aus einer zumeist wässrigen Außenlösung oder auch einer Gasphase in der Innenelektrolytraum des Sensors. Dieser Innenelektrolytraum enthält neben einer flüssigen oder festen Innenelektrolytschicht auch Elektroden zur elektrochemischen Bestimmung des Gases, insbesondere Mess- oder Arbeitselektroden, Gegenelektroden oder Referenzelektroden. In diesem Innenelektrolytraum laufen die eigentlichen elektrochemischen Nachweisreaktionen zur Bestimmung des Gases mittels amperometrischer oder potentiometrischer Methoden ab.

Auch bei optisch-chemischen Sensoren sind eine Reihe von z.T. sehr unterschiedlichen Bauformen und Messprinzipien bekannt geworden. Im Gegensatz zu elektrochemischen Sensoren benötigen optische Sensoren keine Referenzelektrode. Ein optisch-chemischer Sensor besteht typischerweise aus einer oder mehreren, auf einem transparenten Substrat aufgebrachten Schichten aus anorganischen und/oder organischen, vorzugsweise polymeren Substanzen, wobei zumindest eine Schicht einen Farbstoff enthält dessen optische Eigenschaften (z.B. Absorption, Lumineszenz, etc.) von einem in einem Probenmedium enthaltenen Analyten abhängen.

Ein häufig eingesetzter Gassensor ist beispielsweise der Sauerstoffsensor nach Clark. Hier trennt eine gaspermeable Membran die Innenelektrolytlösung vom wässrigen Außenmedium, dem Messmedium. In die Innenelektrolytlösung tauchen im einfachsten Fall zwei Elektroden ein, von denen eine als Arbeitselektrode dicht hinter der Membran angeordnet ist. Nach Anlegen einer Polarisationsspannung geeigneter Höhe wird der aus dem Messmedium durch die Membran in den Innenelektrolytraum diffundierte Sauerstoff an der Arbeitselektrode durch elektrochemische Reduktion verbraucht und es fließt ein dem Stoffumsatz entsprechender Strom. Dieser Strom ist proportional zum Sauerstoffpartialdruck im Messmedium und stellt die primäre Messgröße dar.

Weitere verbreitete elektrochemische Gassensoren, welche solche gasdurchlässigen Membranen besitzen, sind beispielsweise potentiometrische Sensoren vom Severinghaus-Typ zur Bestimmung von Kohlendioxid oder elektrochemische Sensoren zur Bestimmung von Wasserstoff mittels Oxidation an Platinelektroden.

Solche elektrochemischen Gassensoren werden häufig in medizinischen und diagnostischen Analysesystemen zur Bestimmung von Gaspartialdrücken beziehungsweise von Gaskonzentrationen in Flüssigkeiten eingesetzt. Insbesondere werden solche elektrochemischen Gassensoren in Blutgasanalysatoren eingesetzt, welche in der Diagnostik eine große Rolle spielen. Blutgasanalysatoren weisen oft mehrere hintereinander geschaltete Sensoren für verschiedene Parameter auf. Die Probenflüssigkeit fließt durch diese Sensoren, wobei die Messung häufig im so genannten "Stop-Flow-Verfahren" mit zum Zeitpunkt der Messung ruhender Probe erfolgt. Solche Systeme werden oft im Routinebetrieb in Krankenhäusern, Laboratorien und Praxen eingesetzt, so dass an die darin enthaltenen Sensoren hohe Ansprüche bezüglich Lebensdauer, Genauigkeit und Reproduzierbarkeit gestellt werden.

So werden beispielsweise zur Sauerstoffbestimmung in den OMNI-Analysesystemen der Roche Diagnostics GmbH amperometrische O₂-Sensoren und potentiometrische CO₂-Sensoren eingesetzt. Bei den Sauerstoffsensoren handelt es sich um miniaturisierte Gassensoren vom Clark-Typ. Diese dort eingesetzten Gassensorelemente umfassen neben dem eigentlichen Sensor mit Innenelektrolytraum und den darin befindlichen Elektroden einen Probenkanal zum Transport und zur Bereitstellung der Probe. Zwischen Innenelektrolytraum und Probenkanal befindet sich eine gasdurchlässige und weitgehend ionen- und flüssigkeitsundurchlässige Kunststoff-Membran, welche Innenelektrolytraum und Probenkanal trennt. Die Membran liegt hier in einem mechanisch aufgespannten Zustand vor.

Häufig werden in elektrochemischen Gassensoren dünne Membranen aus Kunststoffen mit Schichtdicken im Mikrometerbereich aus hydrophoben Kunststoffen eingesetzt, insbesondere aus Polytetrafluorethylen, Polypropylen oder Polyethylen. Weitere Angaben zu bevorzugten Membranmaterialien sind in "Measurement of Oxygen by Membrane-covered Probes" (Ellis Horwood series in analytical chemistry), 1988, Ellis Horwood Limited, Seite 91f. zu finden.

Bei der Bestimmung von gasförmigen Analyten in wässrigen Lösungen mittels elektrochemischer Gassensoren, insbesondere in physiologischen Flüssigkeiten, wie beispielsweise Vollblut, Serum oder Harn, können in wenigen seltenen Fällen Probleme bei der Probenmessung bzw. bei der Kalibrierung oder Qualitätskontrolle auftreten, wenn die Probe bzw. das Qualitätskontroll- oder Kalibriermittel den Probenkanal nur unvollständig füllt oder wenn sich in der Lösung im Bereich der Sensoren Gasblasen, beispielsweise Luftblasen, befinden. Insbesondere bei Blutgasanalysesystemen mit Sensorelementen für kleine Probenvolumina können Gasblasen zu Fehlmessungen führen, so dass im Hinblick auf das Vorhandensein oder Fehlen von Gasblasen eine wirksame Überprüfung durchgeführt werden muss. Gasblasen bleiben vorzugsweise an der Membranoberfläche hängen. Dieses Phänomen wird beobachtet, wenn beim Befüllvorgang des Probenkanals die wässrige Flüssigkeit der hydrophoben Oberfläche der Membran ein- oder beidseitig ausweicht. Hat die Flüssigkeitsfront die Möglichkeit, seitlich an der Membran vorbeizulaufen, noch bevor diese vollständig mit der Flüssigkeit bedeckt ist, bildet sich eine Gasblase im Bereich der Membran. Sowohl bereits existierende als auch neu gebildete Gasblasen bleiben bevorzugt an der Membran hängen und werden oft auch durch eine Flüssigkeitsströmung nicht mitgerissen. Eine an der Membran haftende bzw. in unmittelbarer Umgebung der Membran befindliche Gasblase hat eine Fehlmessung zur Folge, die ohne zusätzliche Maßnahmen zur Detektion der Gasblase nicht erkannt wird.

So wird bereits in der US 4,358,423 A (Nedetzky) auf das Problem von eingeschlossenen Luftblasen hingewiesen, welche das Messresultat verfälschen, da die Luftblasen eine ausreichende Benetzung der Oberfläche der jeweils eingesetzten Sensoren verhindern. Maßnahmen, die einen derartigen Fehler erkennen, sind insbesondere bei automatisch arbeitenden Analysatoren notwendig, bei welchen der Füllvorgang der Messkapillare bzw. die Blasenfreiheit der Probe in der Messkammer automatisch kontrolliert werden muss. In der US 4,358,423 wird zur Lösung des Problems ein Verfahren vorgeschlagen, bei welchem der Wert des elektrischen Widerstandes zwischen wenigstens zwei Stellen in der Messkammer gemessen wird, wobei der Füllvorgang der Messkammer in Abhängigkeit der festgestellten Größe des gemessenen Widerstandes gesteuert wird.

Mit dem beschriebenen Verfahren können allerdings Luftblasen, die den Querschnitt des Messkanals bzw. der Messkapillare nur teilweise ausfüllen, nicht wirksam detektiert werden. Die Widerstandsmessung würde in einem derartigen Fall zwar geringfügige Unterschiede im Messsignal aufzeigen, welche allerdings von Signaländerungen basierend auf unterschiedlicher Leitfähigkeit der einzelnen Proben - bedingt durch z.B. unterschiedliche Hämatokritwerte - nicht unterschieden werden können.

Aus der EP 1 394 534 A2 ist Verfahren und eine Vorrichtung zur Überprüfung der Positionierung und der Blasenfreiheit einer medizinischen Mikroprobe in einer Durchflussmesszelle eines Analysators mit Hilfe einer an die Messzelle angelegten Wechselspannung bekannt. Die Messzelle weist mehrere hintereinander angeordnete Elektrodenanordnungen mit jeweils mehreren Einzelelektroden (Arbeits-, Referenz- und Gegenelektrode) zur Messung eines Inhaltsstoffes der Mikroprobe mit Hilfe einer Messspannung (im Wesentlichen eine Gleichspannung) auf. Zur genauen Lokalisation der Mikroprobe bzw. zur Detektion von Luftblasen im Bereich jeder Elektrodenanordnung erfolgt sowohl die Einkopplung der Wechselspannung als auch die Einkopplung der Messspannung direkt und gleichzeitig über die Einzelelektroden der jeweiligen Elektrodenanordnung und der gemessene Wechselstromanteil bzw. die Impedanz ist ein Maß für die Position der Mikroprobe und deren Blasenfreiheit. Nachteilig ist auch hier, dass sehr kleine Luftblasen nicht wirksam detektiert werden können.

Aus der EP 0 484 876 B1 eine Einrichtung zum dynamischen Messen des Blasengehalts einer fließenden Flüssigkeit bekannt, welche eine Einrichtung zum Messen des Druckes, der Temperatur und der volumetrischen Durchflussrate der Flüssigkeit aufweist, wobei die Durchflussrate zwischen Punkten eines hohen und eines niedrigen Druckes gemessen und daraus der Blasengehalt der Flüssigkeit berechnet wird. Das Verfahren nützt im Wesentlichen die aus der Druckänderung resultierende Volumsänderung der Flüssigkeit aus, welche abhängig vom Blasengehalt ist.

Aus der WO 01/33195 A1 ist schließlich ein Verfahren und eine Vorrichtung zur Blasendetektion in einer Flüssigkeit bekannt geworden, bei welcher sich die Flüssigkeit beispielsweise in Kontakt mit einem pO₂- Sensor befindet. Zur Überprüfung, ob eine Gasblase im Bereich des Sensors vorliegt, wird nun bei einem ersten Druckwert in der Messkammer ein erster Messwert für den O₂ Partialdruck durchgeführt und anschließend der Druck auf einen zweiten Druckwert in der Messkammer geändert. Auch bei diesem zweiten Druckwert wird der O₂ Partialdruck gemessen und ein zweiter Messwert erstellt. Dieser zweite Messwert wird mit einem Erwartungswert beim geänderten Druck verglichen und je nach Differenz der beiden Werte auf das Vorhandensein von Gasblasen geschlossen. Bei Druckminderung kommt es zu einer Signalerniedrigung infolge der Verminderung des O₂ Partialdrucks der Gasblase. Bei Druckerhöhung kommt es zu einer entsprechenden Signalerhöhung infolge der Erhöhung des O₂ Partialdrucks der Gasblase. Nachteilig dabei ist die Notwendigkeit der genauen Einstellung zweier Druckwerte in der Messkammer.

Aufgabe der vorliegenden Erfindung ist es, ein Verfahren zur Detektion einer Gasblase, welche sich in zumindest teilweisem Kontakt mit dem sensitiven Bereich eines in einer Messkammer angeordneten Sensors befindet derart zu verbessern, dass auch sehr kleine Gasblasen ohne zusätzliche Um- oder Einbauten in der Messkammer eindeutig detektiert werden können, sodass geeignete Maßnahmen eingeleitet werden können.

Diese Aufgabe wird erfindungsgemäß dadurch gelöst:
- dass am Sensor mittels einer wässrigen Flüssigkeit, eines Gases oder eines Gasgemisches, welche(s) die zu bestimmende Gaskomponente in einer geeigneten Konzentration enthält, ein Gaspartialdruck eingestellt wird, welcher sich vom Erwartungswert des Gaspartialdruckes der Gaskomponente der zu messenden Flüssigkeit unterscheidet,
- dass der Sensor mit der zu messenden Flüssigkeit beaufschlagt und ein Stillstand der Messflüssigkeit abgewartet wird,
- dass bis zur Einstellung eines konstanten Signalverlaufes des Sensors dessen zeitlicher Signalverlauf erfasst wird und
- dass aus dem zeitlichen Signalverlauf auf das Vorliegen oder Fehlen einer Gasblase geschlossen wird.

Das erfindungsgemäße Verfahren nutzt die Tatsache dass Gassensoren, die auf optisch-chemischen oder elektrochemischen Messprinzipien beruhen eine bestimmte Einstellkinetik für das Sensorsignal aufweisen. Dabei wird vorausgesetzt, dass die vom Sensor zu bestimmende Gaskomponente Bestandteil der zu detektierenden Gasblase ist.

So benötigen beispielsweise amperometrische O₂-Sensoren für die Einstellung des Signals, ausgehend von einem ersten Signalwert, der einem voreingestellten pO₂ entspricht, auf einen zweiten Signalwert, der dem pO₂ der Probe entspricht, eine gewisse Zeit. Die Einstellkinetik ist umso langsamer, je größer das O₂-Reservoir des Sensors ist. Das O₂-Reservoir ist abhängig von der Größe des Sensors und der Art der Sensormaterialien in unmittelbarer Umgebung der O₂-sensitiven Elemente.

Das erfindungsgemäße Verfahren nutzt bei Verwendung von amperometrischen O₂-Sensoren zudem die Tatsache, dass diese Sensoren den Analyten (O₂) verbrauchen und die O₂-Verarmung in der Probe abhängig ist vom zeitlichen Angebot an Gasmolekülen in unmittelbarer Umgebung der O₂-sensitiven Elemente des Sensors.

Gemäß einer vorteilhaften Variante der Erfindung wird bei zumindest zwei Zeitpunkten während der Einstellzeit des Sensors ein Signalwert des Signalverlaufs gemessen und mit bekannten Werten blasenfreier Messproben verglichen. Dabei ist es von Vorteil, wenn am Sensor vor der Messung ein Gaspartialdruck eingestellt wird, welcher größer ist als der Gaspartialdruck der Gaskomponente in der zu messenden Flüssigkeit.

Die Einstellung des Gaspartialdruckes am Sensor kann vor der Beaufschlagung mit der zu messenden Flüssigkeit durch eine wässrige Flüssigkeit erfolgen, welche die zu bestimmende Gaskomponente in einer geeigneten Konzentration gelöst enthält, beispielsweise mit einer entsprechend tonometrierten Kalibrier-, Kontroll- oder Waschflüssigkeit.

Es ist auch möglich die Einstellung des Gaspartialdruckes am Sensor vor der Beaufschlagung mit der zu messenden Flüssigkeit durch ein Gas oder Gasgemisch vorzunehmen, welches die zu bestimmende Gaskomponente in einer geeigneten Konzentration enthält.

Die Erfindung wird im Folgenden anhand von schematischen Darstellungen und Diagrammen näher erläutert. Es zeigen:
- Fig. 1: eine Messanordnung zur Durchführung des erfindungsgemäßen Verfahrens;
- Fig. 2: die Rohdaten des zeitlichen Verlaufs der Sensorsignale für ein Messbeispiel mit bzw. ohne Luftblase;
- Fig. 3: die normierten Signalverläufe des Messbeispiels gemäß Fig. 2; sowie
- Fig. 4: den zeitlichen Verlauf der Sensorsignale für unterschiedliche Messbeispiele jeweils mit bzw. ohne Luftblase.

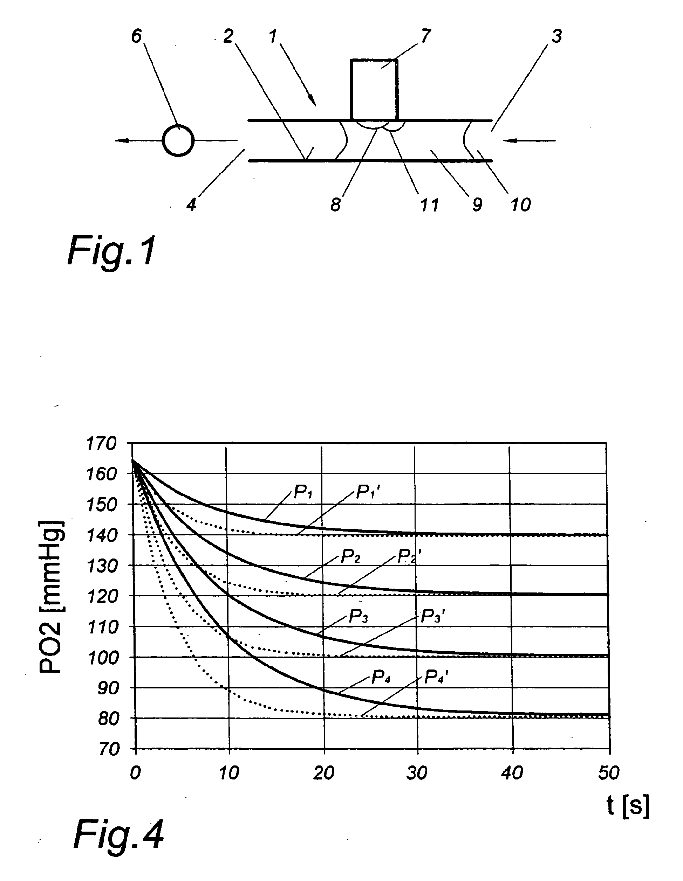

Die in Fig. 1 schematisch dargestellte Messanordnung weist eine Messkammer 1 mit einem durchgehenden Probenkanal 2 auf, dessen Einlassöffnung mit 3 bezeichnet ist. An der Auslassöffnung 4 befindet sich eine Pump- oder Saugeinrichtung 6, beispielsweise eine Peristaltikpumpe, für den Transport der Kalibrier-, Kontroll-, Wasch- oder Probenflüssigkeit. Im Probenkanal 2 können mehrere unterschiedliche, elektrochemische und/oder optisch-chemischen Gassensoren angeordnet sein, dargestellt ist ein amperometrischer Sensor 7, beispielsweise ein O₂-Sensor, dessen sensitiver Bereich 8 in den Probenkanal 2 und in die dort befindliche Messflüssigkeit 9 (z.B. Kalibrier-, Kontroll-, Wasch- oder Probenflüssigkeit) ragt. Die Messflüssigkeit 9 kann einlassseitig durch eine Trenngasblasen 10 von einer nachfolgenden Flüssigkeitsprobe abgegrenzt sein. Eine störende Gasblase 11 steht in zumindest teilweisem Kontakt mit dem sensitiven Bereich 8 des amperometrischen Sensors 7.

Das erfindungsgemäße Verfahren ist besonders geeignet für Messverfahren in biologischen Flüssigkeiten, z.B. zur in-vitro Bestimmung des Blut-pO₂ mittels eines amperometrischen O₂-Sensors (siehe Fig. 1) und wird anhand des folgenden Beispiels näher beschrieben:

Unmittelbar vor der Beaufschlagung des Sensors 7 mit der Messflüssigkeit 9 (z.B. Blutprobe) wird am Sensor ein vorbekannter pO₂ Wert eingestellt. Die Einstellung des pO₂-Wertes erfolgt mit einem flüssigen oder gasförmigen sauerstoffhältigen Medium, (z.B. Luft der Trenngasblase 10 oder entsprechend tonometrierte Waschlösung). Bevorzugt wird ein O₂ Gaspartialdruck eingestellt der sich vom Erwartungswert des Gaspartialdrucks der Messflüssigkeit unterscheidet. Besonders bevorzugt wird ein O₂ Gaspartialdruck eingestellt der größer ist als der Erwartungswert des Gaspartialdrucks der Messflüssigkeit.

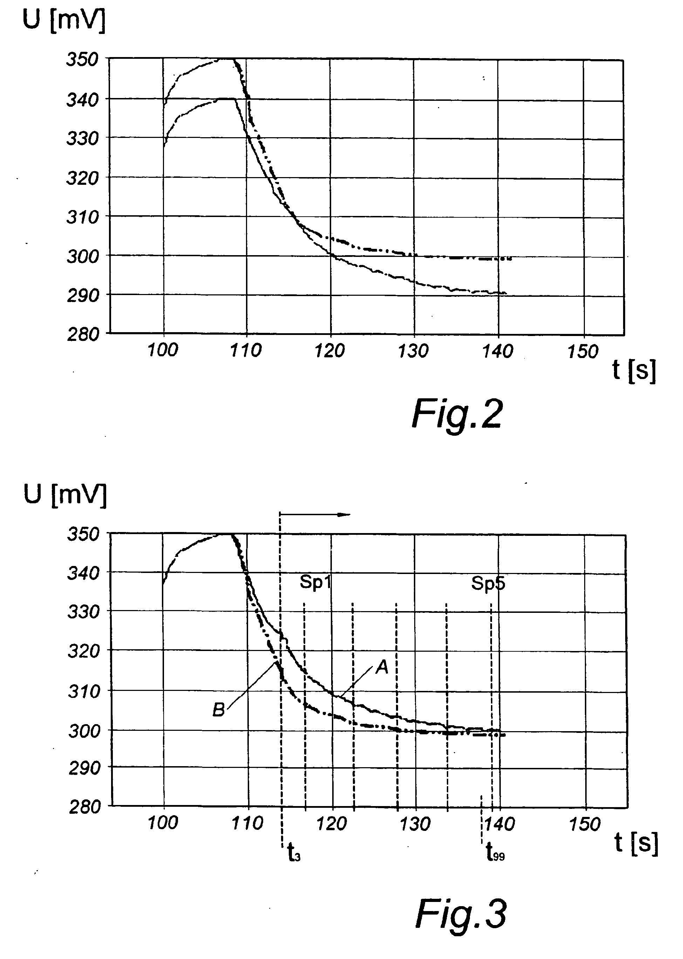

Der Sensor 7 wird mit der Messflüssigkeit 9 beaufschlagt, wobei der Probenkanal 2 mit der Messflüssigkeit 9 gefüllt, gegebenenfalls auch mit der Messflüssigkeit gespült und dann die Bewegung der Messflüssigkeit zum Zeitpunkt tₛ gestoppt wird, so dass sich der Sensor in Kontakt mit der stehenden Messflüssigkeit befindet. Danach gleicht sich der im sensitiven Bereich 8 des Sensors 7 und in der Probe befindliche Sauerstoffpartialdruck durch Diffusionsprozesse aus. Der durch den Ausgleichprozess bedingte Signalverlauf ist in Fig. 2 dargestellt, wobei hier die Rohdaten eines Signalverlaufs ohne Luftblase (volle Linie) bzw. mit Luftblase (strichpunktierte Linie) dargestellt sind. Die Messung kann kontinuierlich erfolgen, es können jedoch auch einzelnen Stützpunktmesswerte Sp1 bis Sp5 (siehe Fig. 3) während der Einstellzeit des Sensors erfasst werden.

Fig. 3 zeigt die normierten Signalverläufe der Messdaten aus Fig. 2, wobei die Messkurven derart normiert wurden, dass die Kurven zum Zeitpunkt t > 150 s zusammen fallen. In den Fig. 2 und Fig. 3 wird auf der Ordinate eine zur O₂ Konzentration der Probe, beispielsweise einer Blutprobe, proportionale Spannung U (in mV) in Abhängigkeit der Zeit t (in s) dargestellt. Dabei wird das amperometrische Signal an einem Strom/Spannungswandler abgegriffen und in Form einer dem Strom proportionalen Spannung gemessen. Die Bewertung der zeitlichen Signalverläufe im Hinblick auf das Vorliegen einer Gasblase kann direkt anhand der gemessenen Spannungswerte erfolgen, eine Umrechnung der Signalverläufe in Druckeinheiten (pO₂ [mmHg]) und die Bewertung auf Basis von Druckeinheiten ist mit Hilfe von Kalibrierdaten zwar möglich aber nicht notwendig.

Wird beispielsweise ein amperometrischer O₂ Sensor mit einem geeigneten Kalibriermedium auf einen pO₂ von ca. 160 Torr eingestellt, und dann mit einer Blutprobe. die einen niedrigeren pO₂ (z.B. 140 Torr) aufweist, in Kontakt gebracht, so beobachtet man über die Zeit t einen exponentiell abnehmenden Signalverlauf. Die Signaländerung (dS/dt) strebt gegen den Wert Null. Unter t₉₉ wird in der Folge die Zeit verstanden, nach der 99% der Signaländerung durchlaufen sind. Das Profil des Signalverlaufs spiegelt die Diffusionsvorgänge des Sauerstoffs vom Sensor in die Probe bzw. von der Probe in den Sensor wieder.

Unter pO₂ Wert des Sensors 7 ist dabei jener pO₂ zu verstehen den die Materialien des Sensors in unmittelbarer Nähe der sensitiven Elemente aufweisen. Unter pO₂ Wert der Probe ist jener pO₂ Wert zu verstehen den die in unmittelbarere Nähe des sensitiven Bereichs 8 des Sensors 7 befindlichen Probenanteile aufweisen.

Bei der Bestimmung des pO₂ Wertes einer Blutprobe mit einem amperometrischen Sensor 7 wurde bei der oben beschriebenen Messsituation überraschend gefunden, dass bei Vorliegen einer Gasblase 11 in direktem Kontakt mit dem sensitiven Bereich 8 des Sensors 7, der Sensor ein schnelleres Einstellverhalten zeigt (siehe Kurve B in Fig. 3) als in Abwesenheit einer Gasblase (siehe Kurve A in Fig. 3). Aus Fig. 3 ist weiters ersichtlich, dass in Anwesenheit der Gasblase t₉₉ schneller erreicht wird. Aus den unterschiedlichen Signalverläufen kann eindeutig auf das Vorhandensein einer Gasblase geschlossen und entsprechende Maßnahmen eingeleitet werden.

Die unterschiedlichen Signalverläufe lassen sich durch unterschiedliche Diffusionseigenschaften, Diffusionswege und Gasmengen in An- bzw. Abwesenheit einer Gasblase erklären. Befindet sich z.B. in unmittelbarer Nähe des Sensors eine Gasblase so ergeben sich im Vergleich zu einer gasblasenfreien Messflüssigkeit unterschiedliche Diffusionsweg und unterschiedliche Gasreservoirs. Innerhalb einer Gasblase sind im allgemeinen die Diffusionskinetiken schneller als innerhalb einer wässrigen Flüssigkeit. Im Allgemeinen enthält eine Gasblase mehr Gasmenge als eine wässrige Flüssigkeit gleichen Volumens.

In Fig. 4 werden die Signalverläufe anhand unterschiedlicher Messwerte dargestellt, wobei mit Hilfe von Kalibrierdaten eine Umrechnung der Spannungswerte in Partialdrücke (pO₂ mmHg) erfolgt. Die einzelnen Kurven von oben nach unten zeigen:
- P₁:: Probe 140 mmHg
- P₁':: Probe 100 mmHg durch Luftblase verfälscht auf 140
- P₂:: Probe 120 mmHg
- P₂':: Probe 70 mmHg durch Luftblase verfälscht auf 120
- P₃:: Probe 100 mmHg
- P₃':: Probe 40 mmHg durch Luftblase verfälscht auf 100
- P₄:: Probe 80 mmHg
- P₄':: Probe 10 mmHg durch Luftblase verfälscht auf 80.

## Patentansprüche

1. Verfahren zur Detektion einer Gasblase, vorzugsweise einer Luftblase, in einer wässrigen Flüssigkeit, vorzugsweise einer Kalibrier-, Kontroll- oder Probenflüssigkeit, welche Gasblase sich in zumindest teilweisem Kontakt mit dem sensitiven Bereich eines in einer Messkammer angeordneten Sensors zur Bestimmung der Konzentration einer in der Flüssigkeit gelösten Gaskomponente befindet, **dadurch gekennzeichnet,**
- **dass** am Sensor mittels einer wässrigen Flüssigkeit, eines Gases oder eines Gasgemisches, welche(s) die zu bestimmende Gaskomponente in einer geeigneten Konzentration enthält, ein Gaspartialdruck eingestellt wird, welcher sich vom Erwartungswert des Gaspartialdruckes der Gaskomponente der zu messenden Flüssigkeit unterscheidet,
- **dass** der Sensor mit der zu messenden Flüssigkeit beaufschlagt und ein Stillstand der Messflüssigkeit abgewartet wird,
- **dass** bis zur Einstellung eines konstanten Signalverlaufes des Sensors dessen zeitlicher Signalverlauf erfasst wird und
- **dass** aus dem zeitlichen Signalverlauf auf das Vorliegen oder Fehlen einer Gasblase geschlossen wird.

2. Verfahren nach Anspruch 1, **dadurch gekennzeichnet, dass** bei zumindest zwei Zeitpunkten während der Einstellzeit des Sensors ein Signalwert des Signalverlaufs gemessen und mit bekannten Werten blasenfreier Messproben verglichen wird.

3. Verfahren nach Anspruch 1 oder 2, **dadurch gekennzeichnet, dass** am Sensor vor der Messung ein Gaspartialdruck eingestellt wird, welcher grö-βer ist als der Gaspartialdruck der Gaskomponente in der zu messenden Flüssigkeit.

4. Verfahren nach Anspruch 1 bis 3, **dadurch gekennzeichnet, dass** die wässrige Flüssigkeit zur Einstellung des Gaspartialdruckes eine entsprechend tonometrierte Kalibrier-, Kontroll- oder Waschflüssigkeit ist.

5. Verfahren nach einem der Ansprüche 1 bis 4, **dadurch gekennzeichnet, dass** als Sensor ein O₂- oder CO₂-Sensor verwendet wird.

6. Verfahren nach einem der Ansprüche 1 bis 5, **dadurch gekennzeichnet, dass** die Probenflüssigkeit eine biologische Flüssigkeit, vorzugsweise Blut, ist.

## Claims

1. Method for detecting a gas bubble, preferably an air bubble, in an aqueous liquid, preferably a calibrating, control or sample fluid, which gas bubble is in at least partial contact with the sensitive region of a sensor which is placed in a measuring chamber and is designed for determining the concentration of a gaseous component dissolved in the liquid, **characterised in that**
- a gas partial pressure, which differs from the expected value of the gas partial pressure of the gas component in the liquid to be measured, is set at the sensor by means of an aqueous liquid, a gas or a mixture of gases containing the gas component to be determined in suitable concentration,
- the sensor is exposed to the liquid to be measured and the liquid is allowed to rest until standstill is attained,
- the sensor signal as a function of time is recorded until the signal becomes constant,
- the presence or absence of a gas bubble is inferred from the variation of the signal over time.

2. Method according to claim 1, **characterised in that** a signal curve value is measured for at least two points in time during the response time of the sensor, and is compared to known values obtained from bubble-free samples.

3. Method according to claim 1 or 2, **characterised in that** a gas partial pressure is set at the sensor before measuring, which is greater than the gas partial pressure of the gas component in the liquid to be measured.

4. Method according to any of claims 1 to 3, **characterised in that** the aqueous liquid used for setting the gas partial pressure at the sensor is a suitably tonometered calibrating, control or rinsing fluid.

5. Method according to any of claims 1 to 4, **characterised in that** the sensor employed is an O₂ or CO₂ sensor.

6. Method according to any of claims 1 to 5, **characterised in that** the sample fluid is a biological fluid, and preferentially blood.

## Revendications

1. Procédé pour la détection d'une bulle gazeuse, de préférence une bulle d'air, dans un liquide aqueux, de préférence un liquide de calibrage, de contrôle ou d'échantillon, laquelle bulle gazeuse se trouve dans un contact au moins partiel avec la zone sensible d'un capteur disposé dans une chambre de mesure pour la détermination de la concentration d'un composant de gaz dissous dans le liquide, **caractérisé en ce que**
◆ une pression partielle de gaz, qui se différencie de la valeur empirique de la pression partielle de gaz du composant de gaz du liquide à mesurer, est réglée sur le capteur au moyen d'un liquide aqueux, d'un gaz ou d'un mélange de gaz, qui contient le composant de gaz àdéterminer dans une concentration appropriée,
◆ **en ce que** le capteur est sollicité avec le liquide à mesurer et un arrêt du liquide de mesure est attendu,
◆ **en ce que**, jusqu'au réglage d'un tracé de signal constant du capteur, son évolution dans le temps est enregistrée et,
◆ à partir de l'évolution du signal dans le temps, on conclut à la présence ou à l'absence d'une bulle de gaz.

2. Procédé selon la revendication 1, **caractérisé en ce que** une valeur de signal de la courbe de signal est mesurée à au moins deux moments pendant le temps de réglage du capteur et comparée avec des valeurs connues d'échantillons de mesure sans bulle.

3. Procédé selon la revendication 1 ou 2, **caractérisé en ce qu'**une pression partielle de gaz, qui est supérieure à la pression partielle de gaz du composant de gaz dans le liquide à mesurer, est réglée sur le capteur avant la mesure.

4. Procédé selon les revendications 1 à 3, **caractérisé en ce que** le liquide aqueux pour le réglage de la pression partielle de gaz est un liquide de calibrage, de contrôle ou de lavage tonométré de façon appropriée.

5. Procédé selon l'une quelconque des revendications 1 à 4, **caractérisé en qu'**un capteur de O₂ ou de CO₂ est utilisé comme capteur.

6. Procédé selon l'une quelconque des revendications 1 à 5, **caractérisé en ce que** l'échantillon de liquide est un liquide biologique, de préférence du sang.
